# EUROPEAN PATENT APPLICATION

(11) **EP 0 674 907 A2**
(43) Date of publication of application: **04.10.1995**
(21) Application number: 95810166.9
(22) Date of filing: 14.03.1995
(51) Int. Cl.: A61K 47/48

(54) **Conjugabes of a antibody and a carrier protein, useful for active immunotherapy**

(30) Priority: 21.03.1994 EP 94810173
(71) Applicant: CIBA-GEIGY AG, CH-4002 Basel (CH)
(72) Inventor: Murray, Brendan, Dr., CH-4416 Bubendorf (CH); O'Reilly, Terence, Dr., D-4057 Basle (CH); Pluschke, Gerd, Dr., CH-79249 Merzhausen (CH)

(57) **Abstract**

The invention relates to a soluble conjugate wherein a carrier protein is covalently linked to a monoclonal antibody, a pharmaceutical preparation comprising such a conjugate and a method for the preparation thereof. The conjugate of the invention is useful e.g. for active immunotherapy of a mammal, particularly a human.

In a preferred embodiment of the invention the conjugate comprises CRM197 and an antibody containing the light chain and/or heavy chain variable regions of the antibody designated MK2-CHγl or the antibody designated MK2-23.

## Description

The present invention relates to a soluble conjugate of a monoclonal antibody and a carrier protein. The present invention further provides a method for preparing such an antibody-carrier protein conjugate, a pharmaceutical preparation comprising said conjugate and a method for evoking an antibody response in a mammal.

A monoclonal anti-idiotypic antibody is an antibody reactive with an antibody variable region epitope (idiotope). Therefore, an anti-idiotypic antibody is often designated as Ab 2 (antibody 2) while the immunizing antibody used for the production of Ab 2 is referred to as Ab 1 (antibody 1). Since an antibody (Ab 1) has multiple idiotopes, some associated with its antigen binding site (paratopes) and others associated with framework determinants of the variable region domain, the complementary antibody response thereto is functionally and structurally heterogeneous and can be divided into three major categories (H.C.J. Ertl and C.A. Bona, Vaccine 6, 80-84 (1988)). One of these categories refers to Ab 2 recognizing a paratope-associated idiotope as Ab 2β. An antibody of this category carries the internal image of the antigen to Ab 1 and therefore is commonly designated as internal image-type (anti-idiotypic) antibody. Criteria distinguishing an internal image-type anti-idiotypic antibody from a "normal", i.e. non internal image-type anti-idiotypic antibody, include immunochemical criteria, such as inhibition of binding of Ab 2 to Ab 1 by antigen, and functional criteria, e.g. induction of an antigen-specific immune response in a mammal which has never before encountered the antigen (H.C.J. Ertl and C.A. Bona, supra). Based on its ability to mimic an antigen, an internal image antibody is capable of inducing in a variety of systems antigen-specific antibodies, also referred to as anti-anti-idiotypic antibodies or Ab 3 (antibody 3). Systems wherein an Ab 3 response may be engendered are e.g. humans and animal test systems, such as mice and goats.

Anti-idiotype-based approaches include the use of an idiotype for passive immunotherapy of T-cell leukemias or B-cell lymphomas (S. Müller, H. Köhler, D. Anderson in Recombinant DNA vaccines: Rationale and Strategy, R.E. Isaacson (ed.), Marcel Dekker, New York, 1992, p. 345-346) and the use of an anti-idiotypic monoclonal, e.g. as a vaccine to induce immunity to a viral, parasitic or bacterial pathogen, as cancer immunotherapeutic agent, as therapeutic agent in immunregulatory disorders and as agonistlantagonist in antigen-antibody binding studies (e.g. J.R. Schreiber, Current Drugs Ltd., pp. 1641-1648 (1993); H.C.J. Ertl and C.A. Bona, supra). For example, an internal image anti-idiotypic antibody may be used as a surrogate antigen and therefore holds great promise for vaccination against an antigen to which there is a suitably reactive antibody available, but the antigen is either poorly defined or unable to be purified. Furthermore, use of an internal image antibody may be appropriate in cases where the antigen itself is unable to induce a suitable immune response, e.g. in the case of certain carbohydrate antigens which do not induce memory responses. An internal image antibody may also be useful for breaking tolerance to a "self" antigen, and therefore is considered a suitable immunological approach for tumor therapy, e.g. melanoma therapy.

Potent immunogenicity of such antibody vaccines is a requirement, and this may demand e.g. formulation with a carrier protein to invoke a T-cell dependent (memory) response. According to the present state of the art aggregated and/or poorly water-soluble or water-insoluble conjugates of an antibody with a carrier protein, such as keyhole limpet haemocyanin (KLH, molecular weight above 1,000,000 Da), are applied to yield an anti-idiotypic (Ab 2) or anti-anti-idiotypic (Ab 3) immune response (see e.g. M. Kusama et al., J. Immunology 143, 3844-3852 (1989); V.K. Lee et al., Biochim. Biophys. Acta 865, 127-139 (1986)); A. Mittelman et al., Proc. Natl. Acad. Sci. USA 89, 466-470 (1992); M. McNamara et al., Science 226, 1325-1326)). Conventional views indicate that aggregates of proteins invoke a more potent immune response than do soluble proteins (e.g. Harlow and Lane (eds), Antibodies, A Laboratory Manual, Cold Spring Harbor Laboratory, 1988, p. 100-101). Typically, random conjugation involving protein aggregation is achieved by means of a homobifunctional crosslinker, such as glutaraldehyde or disuccinimidyl suberate.

However, with respect to clinical use aggregated and/or insoluble antibody-protein conjugates have disadvantages, e.g. the disadvantage of being a rather heterogeneous, hardly standardizable preparation. Thus there is a need for an antibody based preparation with improved properties, e.g. a more uniform preparation which would facilitate standardization of the commercial product.

It is the object of the present invention to meet this need by providing an antibody-carrier protein conjugate which is soluble and non-aggregated and has improved properties for clinical use.

Surprisingly, it has been found that such a soluble, non-aggregated conjugate possesses potent antigenicity which upon in vivo administration results in an appropriate anti-idiotypic (Ab 2) or anti-anti-idiotypic (Ab 3) antibody response directed against the original antigen. On in vivo testing of the soluble conjugates of the invention the specific anti-idiotope humoral immune response is at least comparable to that induced by commonly used aggregates. Preferably, the immunogenicity of the soluble conjugates of the invention comprising a particular antibody, e.g. an anti-idiotypic antibody, is superior to that of poorly soluble or insoluble (in water-based solutions) aggregates comprising the same particular antibody. This superior antigenicity results in a higher antigen specific antibody (Ab 2 or Ab 3) titer.

Furthermore, the present invention provides a method for the preparation of such conjugate, a pharmaceutical preparation comprising such conjugate and a therapeutical method comprising administration of such conjugate to a mammal.

More specifically, the present invention relates to a defined, soluble conjugate wherein a monoclonal antibody molecule, preferably an anti-idiotypic antibody molecule, is covalently linked to at least one carrier protein molecule. The conjugate of the invention is capable of inducing an antibody response (Ab 2 or Ab 3 response) in a mammal. The conjugation of the components is targeted in that the coupling of the carrier protein is restricted to the constant region part of the antibody. To this end, the coupling method is chosen such that it will only involve suitable groups in the constant regions which are not available or accessible in the variable regions. For example, such a directional site-selective conjugation to the antibody may be achieved by suitable activation of the carrier protein, e.g. by reaction with a heterobifunctional crosslinker, e.g. such a crosslinker comprising an N-hydroxysuccinimide (NHS) end, and subsequent reaction of the activated carrier protein with the antibody. Conjugation of the antibody to the carrier protein must not affect the conformation and/or accessibility of the variable regions of the antibody.

Furthermore, the conjugate of the invention is defined in that
- it comprises only one antibody molecule to which a limited number of carrier protein molecules is covalently linked;
- the final product conjugate itself is non-aggregated.

As used herein, "soluble" means that the conjugate of the invention is soluble in water or a water-based solution such as a suitable buffer, e.g. a buffer in which the conjugate components are also soluble individually.

In the conjugate of the invention, the antibody component may be derivable from an antibody which in its cell-bound form serves as unique tumor-specific antigen, e.g. an antibody associated with a B-cell lymphoma. Preferably, the antibody component is an anti-idiotypic antibody, particularly a monoclonal internal image-type antibody (Ab 2β), i.e. the antibody component is reactive with antigen-binding structures on the immunizing antibody (Ab 1) which are complementary to the antigen. Such an antibody represents the conformational mirror image of the antigen and can be used as antigen surrogate. Thus, a conjugate of the invention comprising an internal image anti-idiotypic antibody will in vitro inhibit the binding of the immunizing antibody to target cells, and is capable of eliciting in vivo anti-anti-idiotypic antibodies (Ab 3), which are directed against the antigen and have the same reactivity pattern as Ab 1. The anti-idiotype may be of mammalian, e.g. murine, origin.

An anti-idiotype suitable for the purpose of the present invention is e.g. an antibody which mimics a viral, fungal, parasitic, bacterial or mammalian, particularly human, antigen. The advantages of a monoclonal anti-idiotype as viral vaccine include the avoidance of potentially pathogenic virus-derived antigens, as well as avoidance of genomic material from attenuated live viruses that could revert to wild type or transform host cells. Suitable viral pathogens include e.g. sendai virus, herpes virus, such as cytomegalovirus (CMV), human immunodeficiency virus (HIV), polio, influenza A, SV 40, retrovirus, and virus-derived antigens, such as the hepatitic B surface antigen (HBsAg). It has been shown that anti-idiotypic antibodies can mimic essential bacterial or parasite antigens and induce resistance in animals against serious or lethal bacterial or parasitic infections, e.g. infections caused by Streptococcus pneumoniae, Escherichia coli, Pseudomonas aeruginosa, meningococcus, trypanosoma or Schistosoma mansoni.

Preferred is a conjugate of the invention suitable as a vaccine, e.g. a conjugate comprising an anti-idiotype component mimicking an epitope on human lymphocyte antigen (HLA; e.g. European patent application EP-A-0 498 767), preferably as a tumor vaccine, e.g. a conjugate comprising an anti-idiotype component mimicking an epitope on carcinoembryonic antigen (CEA) or a tumor associated antigen (TAA), particularly, a melanoma associated antigen (cf. e.g. S. Müller, H. Köhler, D. Anderson, supra, p. 343-351). Melanoma associated antigens are e.g. p97 (e.g. M. Kahn et al., Cancer Res. 49, 3157-3162 (1989)) and high molecular weight-melanoma associated antigen (HMW-MAA) (e.g. European patent application EP-A-0 428 485 and international application WO 89/11296). In a particularly preferred embodiment of the invention the antibody component of the conjugate is an internal image of determinants recognized by a monoclonal antibody on HMW-MAA, e.g. by the monoclonal antibody designated MAb 763.74 (P. Giacomini et al., J. Immunol. 135, 696 (1985)). Examples for such particularly preferred conjugates are molecules comprising as antibody component murine anti-idiotypic monoclonal antibody MK2-23 (European patent application EP-A-0 428 485 which is incorporated herein by reference), or an antibody derivable therefrom, e.g. an isotype switch variant thereof, or a chimaeric antibody, e.g. MK2-CHγl (international patent application WO 93/19180 which is incorporated herein by reference). Antibody MK2-CHγl comprises light chain human constant regions kappa (κ), heavy chain human constant regions γ and variable regions derivable from murine antiidiotypic monoclonal antibody MK2-23. The amino acid sequences of the heavy chain and light chain variable domains of antibody MK2-CHγl are set forth SEQ ID NOs. 1 and 3, respectively. MK2-CHγl is produced by the hybridoma cell line MK-CHγl-6, which has been deposited with the European Collection Of Animal Cell Cultures (ECACC), Porton Down, Salisbury, Wilts SP40JG, United Kingdom, on March 6, 1992 under the accession number 92030462.

The antibody component of a preferred conjugate of the invention comprises a heavy chain variable domain comprising a polypeptide of the amino acid sequence set forth in SEQ ID No. 1, or a polypeptide of the amino acid sequence set forth in SEQ ID No. 2.

From reading the present disclosure it will be evident to those skilled in the art that in lieu of a mammalian, e.g. a murine, monoclonal antibody, or a chimaeric monoclonal antibody wherein the variable and constant domains are derived from different species, a fragment of such an antibody, e.g. a F(ab′)₂ fragment, or, if appropriate, a reshaped (humanized) antibody may be employed to form a conjugate of the present invention.

The antibody may be of any immunoglobulin class/subclass, preferentially of class IgG.

As carrier protein component, the conjugate of the invention may comprise any carrier protein useful in humans, e.g a non-toxic, nonpyrogenic, water soluble, pharmaceutically acceptable carrier protein, polypeptide or oligopeptide, preferably such a proteinacoeus molecule having exposed amino groups, e.g. a mutant diphteria toxin devoid of toxin activity (Uchida et al., J. Biol. Chem. 218, 3838-3844 (1973)). Suitable is any proteinaceous molecule which contains highly immunogenic promiscuous T-cell epitopes which will bind to a broad range of polymorphic HLA class I and class II gene products, e.g. a microbial, particularly a bacterial, protein, polypeptide or oligopeptide.

Particularly preferred is a carrier protein component obtainable from a bacterial toxin, e.g. tetanus toxin (see e.g. B. Bizzini in Bacterial Vaccines, Academic Press, 1984: Tetanus, pp. 38-68) or diphteria toxin, which protein component is devoid of toxin activity, but retains the antigenic properties particular to the toxin, e.g. potent immunogenicity. Such non-toxic carrier protein component is obtainable e.g. by detoxification of the toxin, e.g. in case of tetanus toxin, or by mutation, e.g. in case of diphteria toxin.

Detoxification may be accomplished chemically according to a method known in the art, e.g. by formaldehyde (formalin) treatment of the crude or purified toxin under conditions known to produce toxoid. For example, a toxin may be toxoided by the addition of formalin to the toxin solution and incubation at about 35° to about 37°C for about 1 month. The toxoid should pass all requirements set forth by the World Health Organization for toxoid for human use. A preferred carrier protein to form a conjugate of the present invention is the tetanus toxoid.

Diphteria toxin is obtainable from culture supernatants of Corynebacterium diphteriae PW8 according to the method disclosed by R.K. Holmes, Infect. Immun. 12, 1392 (1975). A preferred carrier protein to form a conjugate of the present invention is an atoxic mutant diphteria toxin, particularly the mutant diphteria toxin CRM197. CRM197 is an atoxic protein which crossreacts immunologically with diphteria toxin and is obtainable from culture supernatants of C. diphteriae C7 (R.K. Holmes, supra; U.S. Patent No. 4,925,792, which are incorporated herein by reference). The CRM197 protein has the same molecular weight as the diphteric toxin and is composed of a fragment B which is identical as to its function and structure to those of the toxin, and of a fragment A, which is nontoxic and differs from the original fragment by one amino acid.

In a conjugate of the present invention at least about one, preferably about one to about five, i.e. about one, about two, about three, about four or about five, carrier protein molecules are covalently linked to one antibody molecule. Preferred is a carrier protein-antibody conjugate wherein the molar ratio is about three to one. The above ratios of carrier protein to antibody of about 1 to 5 carrier protein molecules per antibody are to be understood as average ratios.

The average number of carrier protein molecules attached to one antibody molecule may be determined by methods known in the art, e.g. by way of determining the molecular weight of the conjugate. From the molecular weight of the conjugate said average number of carrier proteins may be calculated as follows: the difference between the molecular weight as determined for the conjugate, e.g. by gel filtration or free flow fractionation, and the molecular weight of the antibody component, e.g. about 150 kiloDalton (kDa) for IgG, is divided by the molecular weight of the carrier protein, e.g. about 50 kDa for CRM197, thus yielding the average number of carrier protein molecules.

For conjugates of the invention comprising an IgG and CRM197, the preferred molecular weight is from about 200 kDa to about 400 kDa, particularly about 300 kDa.

The carrier protein is covalently attached to the antibody via a linkage involving selected functional groups of the antibody. Typically, a linkage involving an amino group of the antibody would not be considered to be suitable for the purpose of the present invention. Advantageously, a carrier protein molecule may be covalently bound to an antibody molecule via a link involving e.g. a rare carbohydrate structure of the antibody, a link involving a functional group of the C-terminal amino acid, particularly the carboxy group, a disulphide link, or most preferably, a thioether link.

Preferentially, the antibody is reacted in a purified form. Purification of the antibody may be achieved by methods known in the art, e.g. a procedure comprising one or more chromatography steps such as affinity chromatography, particularly chromatography on Protein A or Protein G affinity media, ion exchange chromatography, hydrophobic interaction chromatography or gel filtration, or a precipitation step, such as ammonium sulphate precipitation.

If appropriate, e.g. in order to facilitate purification, a particular antibody may be subjected to an isotype switch. Such an isotype switch variant of a particular antibody may be produced according to conventional methods, e.g. matched sets of Ig with structurally identical variable regions but different heavy chain constant regions can be obtained by the isolation of hybridoma Ig heavy chain class switch variants which occur spontaneously at frequencies between about 10⁻⁴ and about 10⁻¹⁰ in growing cultures. For example, instead of using murine antibody MK2-23 which is a G1 type immunoglobulin it may be advantageous to use a G2a or G2b type switch variant because contrary to the IgG1 antibody this variant can be affinity purified on Protein A or Protein G affinity media. To isolate such an IgG1 to IgG2a or IgG1 to IgG2b heavy chain isotype switch variant of the hybridoma MK2-23, sequential sublining may be used in combination with an IgG2a- or IgG2b-specific ELISA.

In one aspect, the present invention relates to an antiidiotypic antibody, e.g. a murine IgG2b antiidiotypic antibody bearing an internal image of an epitope of the HMW-MAA, conjugated to a mutated non-toxic diphteria toxin, e.g. CRM 197, or tetanus toxoid.

In a preferred embodiment of the invention the conjugate comprises CRM197 or tetanus toxoid as carrier protein component and an antibody component comprising the light chain and/or heavy chain variable regions of the antibody designated MK2-CHγl or the antibody designated MK2-23.

More preferred is a conjugate comprising CRM 197, or tetanus toxoid, as carrier protein component and an IgG 2-type antibody component comprising the heavy chain variable domain having the amino acid sequence set forth in SEQ ID No. 2 and the light chain variable domain having the amino acid sequence set forth in SEQ ID No. 3.

Particularly preferred is the conjugate comprising an antiidiotypic anti-HMW-MAA murine Ig2b:kappa monoclonal antibody characterized by heavy chain variable domains having the amino acid sequence set forth in SEQ ID No. 2 and by light chain variable domains having the amino acid sequence set forth in SEQ ID No. 3, and the mutated diphteria toxin CRM 197.

The coupling reaction is performed according to methods known in the art in such a way that aggregation is avoided, the antibody is not denatured and non-covalent bonds in the antibody are essentially maintained. The chemistry should be chosen such that the formation of unwanted side products (e.g. homoconjugates) is minimized while the yield of functionally active conjugate is maximized. Moreover, the conjugate should be stable and well defined with respect to the actual linking functions. A procedure using a hetero-bifunctional coupling agent is preferred. Since heterobifunctional crosslinkers possess two selectively reactive groups which can be used to link proteins in a stepwise and specific manner, the occurrence of unwanted side reactions such as the formation of homoproteins is desirably avoided. The number of carrier protein molecules being attached to each antibody molecule may be controlled by varying the molar input ratio of the reaction partners. Attachment of the carrier protein may be across the H and L chain constant regions of the antibody.

A link involving a carbohydrate structure of the antibody may be formed according to methods known in the art (cf. e.g. D.J. O'Shannessy, R. H. Quarles, J. Immunological Methods 99, 153-161(1987)), e.g. by mild oxidation and subsequent modification of the carbohydrate groups. For example, such a conjugate of the invention may be formed by means of a coupling agent having a hydrazide moiety.

A linkage involving a C-terminal carboxy group of the antibody is effected by conventional methods, e.g. a method disclosed in U.S. Patent No. 5,234, 820.

Coupling of the carrier protein to the antibody may involve activation of the carrier protein, e.g. tetanus toxoid or CRM197, by reacting it with a suitable heterobifunctional coupling agent. A suitable heterobifunctional coupling agent has two different reactive groups, one of which is capable of reacting with a functional group of the carrier protein, particularly an amino group of the carrier protein. Preferably, said reactive group in the coupling agent is a N-hydroxysuccinimidester moiety.

Attachment of a carrier protein molecule to an antibody molecule via disulphide or thioether linkage requires at least one accessible thiol (sulfydryl) group in the antibody. Directional site-selective conjugation to the antibody may be effected through reaction of the activated carrier with hinge and interchain cysteins of the antibody, taking advantage of the thiol groups released by reduction of interchain disulphide bonds. Such reduction is performed under mild conditions, e.g. conditions not involving gross unfolding of the protein, using a suitable reducing agent. A suitable reducing agent is e.g. an agent which if employed in sufficient molar surplus (over the antibody) favors the completion of the disulphide interchange, e.g. a dithiol, such as dithiothreitol (DTT). According to their susceptibility to disulphide interchange the disulphide bonds in an antibody can be divided into three categories: shielded bonds (usually intrachain), assisted bonds and unassisted bonds (both usually interchain) (G.T. Stevenson et al. in Protein Engineering of Antibody Molecules for Prophylactic and Therapeutic Applications in Man, ed. M. Clark, Academic Titles, Bramcote, Nottingham, U.K., 1993, 127-141). For the preparation of a conjugate of the invention reduction of the latter type is the most suitable. Preparation of a conjugate of the invention involving one or more disulphide or thioether linkages requires that at least one disulphide bond in the antibody is reduced.

For example, a heterobifunctional lysine/cysteine crosslinking agent, such as sulfosuccinimidyl-4-(p-maleimidphenyl) butyrate (sulfo-SMPB), may be used to attach the carrier protein to the antibody. The carrier protein is reacted with such a crosslinking agent via accessible amino acid residues that bear a reactive primary amine, such as surface-exposed lysine residues, thus forming an activated carrier protein capable of binding to reactive thiols. This activated carrier protein bearing surface exposed cysteine reactive moieties is then exposed to antibody that has been partially reduced, e.g. with dithiothreitol. The partial reduction conditions expose some, but not all, of the disulfide bonds present in the hinge region of the antibody. The conjugates may then be purified and analysed according to methods known in the art.

Suitable heterobifunctional coupling agents are commercially available or can be prepared according to methods known in the art and include e.g. crosslinkers containing an amine reactive group, e.g. N-hydroxysuccinimide esters, and a sulfhydryl reactive group, e.g. a haloacetyl function, a maleimide function, or a reactive disulphide, such as N-(3-[2'pyridyldithio]propionoxy)-succinimide (SPDP), for coupling via disulphide bonds. Agents suitable for alkylation of an antibody thiol group resulting in the formation of a thioether bond include N-succinimidyl bromoacetate or crosslinkers of the maleimide-N-hydroxysuccinimid ester type. Crosslinkers of the maleimide-N-hydroxysuccinimide ester type may be aromatic, such as m-maleimidobenzoyl-N-hydroxysuccinimide ester (MBS), sulfosuccinimidyl 4-(p-maleimidophenyl)butyrate (S-SMPB or Sulfo-SMPB) and m-maleimidobenzoylsulfosuccimide ester (S-MBS), or aliphatic, such as N-γ-maleimidobuturyloxysuccimide ester (GMBS) (D.E. Myers et al., J. Immunological Methods 121, 129-141(1989)). Reaction of the carrier protein with such a coupling agent results in an activated carrier protein which is capable of reacting with the antibody molecule due to the introduction of a reactive group which essentially does not react with the carrier protein. Before reaction of the activated carrier protein with the antibody, excess coupling agent should be removed, e.g. via gel filtration or dialysis.

Linkage of the conjugate components via thioether bonds is the most preferred.

A preferred conjugate of the invention is a soluble, non-aggregated conjugate of an internal image-type antiidiotypic monoclonal antibody, particularly an antiidiotypic antibody which is the internal image of determinants recognized by a monoclonal antibody on HMW-MAA, with the CRM197 protein or tetanus toxoid, which conjugate is characterized in that one antibody molecule carries an average of one to five, preferably an average of three, of said carrier protein molecules which are site-selectively and covalently attached to the constant region part of the antibody via a thioether bond. Particularly preferred is such a conjugate obtained by use of Sulfo-SMPB as a heterobi-functional coupling agent.

The induction of an immune response (Ab3 response), e.g. an anti-HMW-MAA immune response, following vaccination with an anti-idiotypic conjugate of the invention may be analysed according to methods known in the art, e.g. by determination of anti-melanoma cell antibody titres, e.g. using an ELISA, particularly a "live-cell" ELISA procedure. A HMW-MAA positive human melanoma cell line, such as A375met, and a HMW-MAA negative melanoma cell line, such as S-7, may be chosen as cellular substrates. The cells are exposed to dilutions of test sera (Ab 3), or positive and negative (non-HMW-MAA-reactive) control monoclonal antibodies. Cell-bound Ab 3 antibodies may be determined by a suitable enzyme-linked antibody conjugate, e.g. an anti-IgG peroxidase conjugate.

The invention further provides a method for preparing a conjugate of the invention, said method comprising reacting an antibody with a carrier protein under conditions allowing the site-selective, covalent attachment of an average of about one to about five carrier protein molecules per one antibody molecule, and isolating the conjugate of the invention. If appropriate, the carrier protein is activated before reaction with the antibody. The resultant products can be purified to yield conjugates with different ratios of antibody and carrier protein. If desired, the conjugate of invention is subjected to further analysis, e.g. for the purpose of "quality control". Such analysis may involve comparison with the non-conjugated antibody, e.g. according to methods generally known in the art, such as immunoassays, e.g. assays involving immunocytochemistry or flow cytometry, methods aimed at the determination of binding kinetics, e.g. assays for the determination of association or dissociation rates, such as biospecific interaction analysis (U. Jönsson et al., BioTechniques 11, 620-627 (1991)), and the like. Preferred is a process yielding an above captioned preferred conjugate of the invention.

More specifically, for the preparation of a conjugate of the invention wherein the carrier protein, e.g. CRM197, is linked to an antibody via a thioether linkage the reaction is carried out in a two step procedure. The carrier protein, which contains no free cysteins, is first reacted with a crosslinker of the maleinimide-N-hydroxysuccinimid ester type. This will result in the addition of cystein reactive maleinimide groups to the carrier protein surface and no crosslinking of the carrier protein molecules. After removal of excess cross linker, e.g. by gelfiltration or dialysis, the activated carrier protein is then co-incubated with the antibody, e.g. an antibody containing the Ig variable regions of antibody MK-CHγ1 or MK2-23, which has been previously partially reduced, e.g. with dithiothreitol, thus forming free, reactive thiols without disrupting the overall protein structure. The reduced form of the antibody is separated from the unreacted reducing agent. The coupling reaction forms covalent bonds between the activated carrier protein and the reduced antibody, resulting in the formation of the carrier protein-antibody conjugate. In the coupling reaction the molar excess of carrier protein is chosen such that the desired number of carrier protein molecules is coupled to one antibody molecule. For example, to yield a conjugate wherein approximately three CRM197 molecules are linked to one murine IgG molecule, e.g. MK2-23, activated CRM197 is reacted in a five-fold molar excess. Preferably, the conjugate according to the invention is chromatographically purified, and if desired, sterilized, e.g. by filtration under aseptic conditions.

A conjugate of the invention can be used as agonist/antagonist in antigen-antibody binding studies, e.g. for diagnostic purposes, for instance for in vitro (ex vivo) or in vivo qualitative and quantitative determination of antibodies (Ab 1 or Ab 3) directed against the particular antigen the antiidiotypic antibody component of the conjugate of the invention is mimicking, or for therapeutic purposes, e.g. as a vaccine to induce immunity to a viral, parasitic or bacterial pathogen, and for control, prevention, (adjuvant) treatment and monitoring of a tumor, e.g. melanoma.

The invention also relates to the use of a conjugate of the invention as a vaccine, and to a method of vaccinating a mammal, particularly a human, comprising administration of a conjugate of the invention. Such a method is intended to also refer to a method of inducing an anti-tumor response in a mammal comprising administration of a conjugate of the invention comprising a suitable antibody.

The conjugates according to the invention are useful for a number of therapeutic and diagnostic purposes. For example, the advantages of using such conjugates comprising as the antibody component an antiidiotypic monoclonal antibody which is the internal image of determinants recognized by a monoclonal antibody on HMW-MAA instead of the HMW-MAA itself are for instance:
· higher immunogenicity of the conjugate;
· stimulation of B-cell clones normally unresponsive to HMW-MAA by the conjugate;
· possible generation of anti-HMW-MAA T-cell responses;
· elimination of side effects which might be associated with the application of HMW-MAA;
· easy production of large amounts of the conjugate.

Due to their immune-regulatory functions within the idiotype-antiidiotype reaction network the conjugates according to the invention can for example be used to modulate the immune response to a tumour associated antigen, such as HMW-MAA. The modulation is specific and can be directed by the choice of the monoclonal antibody used for the production of the antiidiotypic antibodies, i.e. the specificity of the immunizing MAb (Ab 1), and by the isotype of the antiidiotypic MAbs, since different isotypes of anti-idiotypic antibodies may have different immune-regulatory action. The conjugates of the invention may have immune-regulatory functions such as the stimulation of humoral and cellular immunity.

Consequently, conjugates according to the invention are e.g. useful agents for the control, treatment or adjuvant treatment of a tumor, e.g. melanoma, that is to say that they can be successfully employed e.g. to cause tumor regression and/or prevent tumor recurrence. The antiidiotypic monoclonal antibodies comprised by the conjugates of the invention can be "tailor-made" to mimic specific determinants of the TAA by the adequate choice of the immunizing antibody. Such antiidiotypic antibody-conjugates of the invention are also useful for preventing a tumor by induction of immunity against the determinants of the TAA recognized by the immunizing monoclonal antibody.

The anti-idiotypic antibody-based conjugates of the invention are useful as vaccines for the adjunct treatment of cancer. For example, a conjugate of the invention inducing Ab3 antibodies reactive with HMW-MAA is useful as a vaccine for active immunotherapy of malignant melanoma, e.g. for the adjunct treatment for malignant melanoma of early stage melanoma patients remaining at risk for metastatic disease after surgery for the primary lesion.

The invention also concerns pharmaceutical compositions comprising a conjugate according to the invention. The pharmaceutical compositions comprise, for example, a conjugate of the invention in a therapeutically effective amount together or in admixture with pharmaceutically acceptable, inorganic or organic, solid or liquid carriers. Preferred are pharmaceutical compositions additionally comprising an adjuvant, i.e. an agent further increasing the immune response. Possible adjuvants are Freund's complete adjuvant (emulsion of mineral oil, water, and mycobacterial extracts), Freund's incomplete adjuvant (emulsion of water and oil only), mineral gels, e.g. aluminium hydroxide gels, surface active substances such as lysolecithin, polyanions, peptides, BCG (Bacillus Calmette-Guerin), etc.. Particularly preferred are pharmaceutical compositions comprising a conjugate of the invention and MF59 (international patent application WO 90/14837) as adjuvant, and, optionally, N-acetylmuramyl-L-alanyl-D-isoglutaminyl-L-alanine-2-[1,2-dipalmitoyl-sn-glycero-3-(hydroxyphosphoryloxy]ethylamide (MTP-PE; international patent application WO 90/14837).

Preferred are pharmaceutical compositions for parenteral application. Compositions for intramuscular, subcutaneous or intravenous application are e.g. isotonic aqueous solutions or suspensions, optionally prepared shortly before use from lyophilized or concentrated preparations. The pharmaceutical compositions may be sterilized and contain adjuvants e.g. for conserving, stabilizing, wetting, emulsifying or solubilizing the ingredients, salts for the regulation of the osmotic pressure, buffer and/or compounds regulating the viscosity, e.g. sodium carboxycellulose, dextran, polyvinylpyrrolidine or gelatine. They are prepared by methods known in the art, e.g. by conventional mixing, dissolving or lyophilizing, and contain from approximately 0.01% to approximately 50% of active ingredients. The compositions for injections are processed, filled into ampoules or vials, and sealed under aseptic conditions according to methods known in the art. For example, owing to the solubility in aqueous solutions a conjugate of the invention may be formulated as a "two vial system" with an above adjuvant, e.g. MF59-0.

Preferred is a pharmaceutical composition comprising a conjugate of the invnetion suitable for intramuscular administration in a depot formulation together with an adjuvant.

Also preferred is a pharmaceutical composition comprising a conjugate of the invention which is suitable for mucosal application (H.F. Staats et al., Current Opinion in Immunology 6, 572-583 (1994)), or a stabilized pharmaceutical composition that can be swallowed for oral immunization.

The specific mode of administration and the dosage will be selected by the attending physician taking into account the particulars of the patient, state and type of the disease to be treated, and the like. The therapeutic dose for mammals is between approximately 0,1 µg and 100 µg, preferably between approximately 0.1 µg and approximately 10 µg, per kg body weight depending on the status of the patient and the mode of application. Most preferably, the dose for mammals is between approximately 0.1 µg and approximately 1 µg per kg body weight.

The conjugates according to the invention can also be used for the qualitative and quantitative determination of antibodies directed against a pathogen, e.g. a pathogen mentioned above, or a TAA, such as HMW-MAA. This is especially useful for the monitoring of the success of the treatment with a conjugate of the invention.

For instance, the conjugates of the invention can be used in an assay which relies on the binding interaction between idiotopes of a particular Ab 1 or Ab 3 and the "corresponding" antiidiotypic monoclonal antibody (Ab 2) of the conjugate of the invention. Examples of such assays are radio-, immunoprecipitation, latex agglutination, and hemagglutination immunoassays.

The invention also concerns test kits for the qualitative and quantitative determination of antibodies (Ab 1 or Ab 3) directed against the particular antigen mimicked by the antibody component of the conjugate of the invention comprising a conjugate of the invention and, optionally, other polyclonal or monoclonal antibodies and/or adjuncts.

The invention particularly concerns the embodiments, e.g. conjugates, methods for the preparation thereof and formulations comprising such conjugates as described in the examples.

The following examples illustrate the invention, but do not limit it to any extent.

### Example 1: Conjugation of CRM197 to antibody MK2-CHγl

1.1 Activation of CRM197 with sulfosuccinimidyl 4-(p-maleimidophenyl)butyrate (S-SMPB)
   245 µl of CRM197 (52 mg/ml) in PBS (25 mM sodium phosphate buffer pH 8.2, 150 mM NaCl) are added to 450 µl of PBS/EDTA (25 mM sodium phosphate buffer pH 8.2, 150 mM NaCl, 1 mM EDTA). A fresh solution of S-SMPB is prepared by the addition of 9 mg of S-SMPB to 1 ml of DMSO (dimethyl sulphoxide). Then 200 µl of this solution are added dropwise to the CRM197. The reaction mixture is then incubated on ice for 2 hr.
1.2 Separation of activated CRM197 from unreacted S-SMPB
   An HR10/10 fast desalting column (Pharmacia) is equilibrated and run at room temperature in PBS/EDTA pH 7.2 at 3 ml/min. The activated CRM197 solution is then loaded onto the column. Protein elution is monitored by ultra-violet absorption at 280 nm. The first emerging peak containing the activated CRM197 in the equilibration buffer is collected (ca 2.2 ml). This solution is transferred to ice.
1.3 Reduction of disulphides in antibody MK2-CHγl to yield reactive thiols
   1 ml of MK2-CHγl (12 mg/ml; international application WO 89/11296) in PBS is adjusted to pH 8.0 with 20 µl of 2 M Trizma Base (Sigma). 50 µl of fresh 1 M aqueous DTT (dithiothreitol) is added (50 mM final concentration). The mixture is then incubated at 37°C in a water bath for 30 min.
1.4 Separation of reduced MK2-CHγl from unreacted DTT
   An HR10/10 fast desalting column is equilibrated and run at room temperature in PBS/EDTA pH 7.2 at 3 ml/min. The reduced antibody solution is then loaded onto the column. Protein elution is monitored by ultra-violet absorption at 280 nm. The first emerging peak containing the reduced antibody in the equilibration buffer is collected (ca 2.2 ml). This solution is transferred to ice.
1.5 Coupling of activated CRM197 to reduced antibody
   The reduced antibody solution is stirred continuously at room temperature while the activated CRM197 is added dropwise. The reaction mixture is then returned to ice and reacted overnight. The final solution contains a small amount of precipitate which is removed by centrifugation and filtration through a 0.22 µm membrane filter.

### Example 2: Analysis of the final coniugate of CRM197 and the antibody

2.1 Gel Filtration
   Analysis of the conjugate by Superose 6 (Pharmacia) gel filtration chromatography reveals a peak of average molecular weight 300 kDa (Peak I) followed by a peak of unreacted CRM197. The molecular weight of 300 kDa corresponds to three CRM197 (Mr (molecular weight) 47 kDa) attached to each antibody.
2.2 SDS-PAGE
   Denaturing gel electrophoresis in the presence or absence of reducing reagent (DTT) reveals a large complex of a Mr of more than 150 kDa. There is no evidence of unreacted antibody but a small amount of CRM197 is observed.
2.3 Free Flow Fractionation
   This method separates molecules based on their diffusion coefficients and is an ideal complement to gel filtration since it can confirm the Mr as well as detect large aggregates. For the Peak I eluate obtained in 2.1, the method reveals the absence of large aggregates and an average Mr of 300 kDa.
2.4 ELISA Assay
   An ELISA assay to test the binding activity of the conjugate reveals that the complex is capable of binding to antibody recognized specifically by the un-conjugated antibody MK2-CHγl.

Taken together, the conjugation reaction is found to attach an average of three CRM197 to each MK2-CHγl molecule. The attachment must be across the H and L chains of the antibody since the complex cannot be broken apart by reducing SDS-PAGE.

### Example 3: Conjugation of CRM 197 to antibody MK2-23

Conjugation of CRM 197 to murine antibody MK2-23 (EP-A-0 428 485) is accomplished analogously to the procedure described in Example 1. In the resulting conjugate, three CRM197 molecules are linked per antibody.

### Example 4: Experimental vaccination of mice with the conjugates of Examples 1 and 3

Conjugates are diluted in pyrogen-free saline to a concentration of 600 µg antibody/ml. Equal volumes of conjugate are mixed either with Freund's Complete Adjuvant (FCA), Freund's Incomplete Adjuvant (IFA) or MF59 Adjuvant (Biocine) either containing 100 µg MTP-PE (MF-100) or not (MF59-0). Conjugate corresponding to 15 µg of antibody is intramuscularly injected into Balb/c mice at 0, 14 and 28 days. Blood samples from mice are obtained 0, 28, 56 and 72 days after the first injection. Titres to the high molecular weight-melanoma associated antigen are determined by an ELISA system that uses adherent A375 cells (American Type Culture Collection ATCC CRL 1619) expressing HMW-MAA as a source of antigen. Antibody titres are obtained by reference to a standard curve of Abl (MAb 763.74), the original antibody used to obtain the mouse anti-idiotype, and subsequently chimeric, antibody.
Administration of the specific conjugate of chimeric antibody:CRM197 (1:3, Example 1) results in a satisfactory HWM-MAA specific antibody titre. The conjugate formed by reaction of murine antibody MK2-23 with CRM197 (Example 3) also yields a strong anti-HMW-MAA antibody response in mice. To indeed demonstrate the surprising finding that the superior immunogenicity of the conjugates resides in a soluble molecule, as opposed to a small amount of antigenically-potent aggregrates, the chimaeric antibody:CRM197 (1:3) is further purified by Superose 6 chromatography to remove high molecular weight (> 6 million) components, and to enrich for a protein of the predicted 300 kDa molecular weight. It is found that the immunogenicity resides in the non-aggregrated 300 kDa fraction, representing the 1:3 antibody:CRM197 conjugate.

### Example 5: Pharmaceutical preparation for parental application

10 mg conjugate comprising chimeric antiidiotypic monoclonal antibody MK-CHγl (Example 1) or murine monoclonal antibody MK2-23 (Example 2) are dissolved in 50 ml PBS. The solution is passed through a bacteriological filter, the filtrate divided into 10 equal parts and filled in ampoules under aseptic conditions. The ampoules are preferably stored in the cold, e.g. at 4°C. These pharmaceutical preparation are suitable for injection and are applied as such or in combination with a pharmaceutical preparation containing MF59 (Biocine) and, optionally the muramyl peptide MTP-PE.

### Example 6: Conjugation of Tetanus Toxoid (TT) to IgG 2b-antibody

The antibody designated IgG 2b-antibody is an IgG 2b switch variant obtainable from antibody MK2-23-producing hybridoma by a sequential sublining method. Antibody IgG 2b comprises a heavy chain variable domain having the amino acid sequence set forth in SEQ ID No. 2 and a light chain variable domain having the amino acid sequence set forth in SEQ ID No. 3.
6.1 Activation of TT with sulfosuccinimidyl 4-(p-maleimidophenyl)butyrate (S-SMPB)
   A fresh solution of S-SMPB is prepared by the addition of 11.3 mg of S-SMPB to 1 ml of DMSO (dimethyl sulphoxide). Then 33.8 µl of this solution are added dropwise to 4.4 ml of TT (5.5 mg/ml) in PBS (25 mM sodium phosphate buffer pH 8.2, 150 mM NaCl). The reaction mixture is then incubated on ice for 2 hrs.
6.2 Separation of activated TT from unreacted S-SMPB
   An HR10/10 fast desalting column (Pharmacia) is equilibrated and run at room temperature in PBS/EDTA (25 mM sodium phosphate buffer, 150 mM NaCl, 1 mM EDTA), pH 7.2 at 2 ml/min. Aliquots of the activated TT solution are then loaded onto the column. Protein elution is monitored by ultra-violet absorption at 280 nm. The first emerging peak containing the activated TT in the equilibration buffer is collected (ca 2.2 ml). This solution is transferred to ice.
6.3 Reduction of disulphides in antibody IgG2b to yield reactive thiols
   0.75 ml of IgG2b (6.6 mg/ml) in PBS is reacted with 15 µl of fresh 0.5 M aqueous DTT (dithiothreitol) containing 50 mM EDTA. The mixture is then incubated at 37°C in a water bath for 15 min.
6.4 Separation of reduced antibody IgG2b from unreacted DTT
   An HR10/10 fast desalting column is equilibrated and run at room temperature in PBS/EDTA pH 7.2 at 2 ml/min. The reduced antibody solution is then loaded onto the column. Protein elution is monitored by ultra-violet absorption at 280 nm. The first emerging peak containing the reduced antibody in the equilibration buffer is collected (ca 2.2 ml). This solution is transferred to ice.
6.5 Coupling of activated TT to reduced antibody
   The solution of the activated TT (9 ml) is stirred continuously at room temperature while the reduced antibody (2.5 ml) is added dropwise. The reaction mixture is then returned to ice and reacted overnight. The final solution is dialyzed for 72h against PBS, pH 7.2 and filtered through a 0.22 µm membrane filter.
6.6 SDS-PAGE
   Denaturing gel electrophoresis in the presence of reducing reagent (β-mercaptoethanol) reveals a large complex of a Mr of more than 150 kDa. There is no evidence of unreacted antibody but residual TT is observed.

### Deposition data:

The hybridoma cell-line MK2-CHγl-6 producing antibody MK2-CHγl has been deposited at the European Collection Of Animal Cell Cultures (ECACC), Porton Down, Salisbury, Wilts SP40JG, United Kingdom, on March 6, 1992 under the accession number 92030642.

## Claims

1. A soluble, non-aggregated carrier protein-antibody conjugate inducing an immune response in vivo, wherein one or more carrier protein molecules are site-selectively and covalently linked to the constant region part of one antibody molecule.

2. The conjugate according to claim 1 wherein the carrier protein is derivable from a bacterial toxin.

3. The conjugate according to claim 2, wherein the carrier protein is selected from the group consisting of CRM 197 or tetanus toxoid.

4. The conjugate according to any of claims 1 to 3, wherein the linkage between the carrier protein and the antibody component involves a thioether bond.

5. The conjugate according to any of claims 1 to 4, wherein the antibody is an anti-idiotypic antibody, particularly an anti-idiotypic antibody which is the internal image of determinants recognized by a monoclonal antibody on high molecular weightmelanoma associated antigen (HMW-MAA).

6. The conjugate according to claim 5, wherein the antibody comprises a light chain variable domain having the amino acid sequence set forth in SEQ ID No. 3 or a heavy chain variable domain selected from the group consisting the polypeptide having the amino acid sequence set forth in SEQ ID No. 1 or the polypeptide having the amino acid sequence set forth in SEQ ID No. 2.

7. A conjugate according to any of claims 1 to 6 for use in a diagnostic or therapeutic method practised on the human or animal body.

8. A conjugate according to claim 7 for use as a vaccine in active immunotherapy.

9. A conjugate according to any of claims 1 to 6 for use in the preparation of a pharmaceutical composition.

10. A pharmaceutical composition comprising a conjugate of any of claims 1 to 6, a pharmaceutically acceptable carrier, and optionally an adjuvant.

11. A process for the preparation of a conjugate according to any of claims 1 to 6, said process comprising reacting the antibody with a carrier protein under conditions allowing the covalent attachment of one or more carrier protein molecules to one antibody, and isolating the desired conjugate.

12. A method of evoking an immune response in a mammal, particularly a human, comprising the administration of a soluble, non-aggregated carrier protein-antibody conjugate, wherein one or more carrier protein molecules are site-selectively and covalently linked to the constant region part of one antibody molecule.

13. A method for active immunotherapy of a mammal, particularly a human, comprising the administration of a conjugate of any of claims 1 to 6.

14. A method of evoking an antitumor response in a mammal, particularly a human, in need thereof comprising the administration of a conjugate of any of claims 1 to 6.

15. A method according to claim 14 wherein the antitumor response is directed to a tumor associated antigen (TAA).

16. A method according to claim 15 wherein the antitumor response is directed to high molecular weight-melanoma associated antigen.


SEQUENCE LISTING

(1) GENERAL INFORMATION:

(i) APPLICANT:
(A) NAME: CIBA-GEIGY AG
(B) STREET: Klybeckstr. 141
(C) CITY: Basel
(E) COUNTRY: SCHWEIZ
(F) POSTAL CODE (ZIP): 4002
(G) TELEPHONE: +41 61 69 11 11
(H) TELEFAX: + 41 61 696 79 76
(I) TELEX: 962 991

(ii) TITLE OF INVENTION: Antibody Conjugate With Improved Properties

(iii) NUMBER OF SEQUENCES: 3

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 117 amino acids
(B) TYPE: amino acid
(C) STRANDEDNESS: single
(D) TOPOLOGY: unknown

(ii) MOLECULE TYPE: protein

(xi) SEQUENCE DESCRIPTION: SEQ ID NO: 1:

Leu Gln Glu Ser Gly Gly Gly Leu Val Gln Pro Gly Gly Ser Arg Lys
1               5                   10                  15

Leu Ser Cys Ala Ala Ser Gly Phe Thr Phe Ser Ser Phe Gly Met His
            20                  25                  30

Trp Val Arg Gln Ala Pro Glu Lys Gly Leu Glu Trp Val Ala Tyr Ile
        35                  40                  45

Ser Ser Asp Ser Ser Asn Ile Tyr Tyr Ala Asp Thr Val Lys Gly Arg
    50                  55                  60

Phe Thr Ile Ser Arg Asp Asn Pro Lys Asn Thr Leu Phe Leu Gln Met
65                  70                  75                  80

Thr Ser Leu Arg Ser Glu Asp Thr Ala Met Tyr Tyr Cys Ala Arg Ser
                85                  90                  95

Asn Tyr Val Gly Tyr His Val Arg Trp Tyr Phe Asp Val Trp Gly Gln
            100                 105                 110

Gly Thr Thr Val Thr
        115

(2) INFORMATION FOR SEQ ID NO: 2:

(i) SEQUENCE CHARACTERISTICS:
(A) LENGTH: 120 amino acids
(B) TYPE: amino acid
(C) STRANDEDNESS: single
(D) TOPOLOGY: unknown

(ii) MOLECULE TYPE: protein

(xi) SEQUENCE DESCRIPTION: SEQ ID NO: 2:

Asp Val Val Leu Val Glu Ser Gly Gly Gly Leu Val Gln Pro Gly Gly
1               5                   10                  15

Ser Arg Lys Leu Ser Cys Ala Ala Ser Gly Phe Thr Phe Ser Ser Phe
            20                  25                  30

Gly Met His Trp Val Arg Gln Ala Pro Glu Lys Gly Leu Glu Trp Val
        35                  40                  45

Ala Tyr Ile Ser Ser Asp Ser Ser Asn Ile Tyr Tyr Ala Asp Thr Val
    50                  55                  60

Lys Gly Arg Phe Thr Ile Ser Arg Asp Asn Pro Lys Asn Thr Leu Phe
65                  70                  75                  80

Leu Gln Met Thr Ser Leu Arg Ser Glu Asp Thr Ala Met Tyr Tyr Cys
                85                  90                  95

Ala Arg Ser Asn Tyr Val Gly Tyr His Val Arg Trp Tyr Phe Asp Val
            100                 105                 110

Trp Gly Gln Gly Thr Thr Val Thr
        115                 120

(2) INFORMATION FOR SEQ ID NO: 3:

(i) SEQUENCE CHARACTERISTICS:
(A) LENGTH: 108 amino acids

(B) TYPE: amino acid
(C) STRANDEDNESS: single
(D) TOPOLOGY: unknown

(ii) MOLECULE TYPE: protein

(xi) SEQUENCE DESCRIPTION: SEQ ID NO: 3:

Gln Leu Thr Gln Ser Pro Ala Ser Leu Ala Val Ser Leu Gly Gln Arg
1               5                   10                  15

Val Thr Ile Ser Cys Arg Ala Ser Glu Ser Val Glu Tyr Tyr Gly Ser
            20                  25                  30

Ser Leu Met Gln Trp Tyr Gln Gln Lys Pro Gly Gln Pro Pro Lys Leu
        35                  40                  45

Leu Ile Tyr Ala Ala Ser Asn Val Glu Ser Gly Val Pro Ala Arg Phe
    50                  55                  60

Ser Gly Ser Gly Ser Gly Thr Asp Phe Ser Leu Asn Ile His Pro Val
65                  70                  75                  80

Glu Glu Asp Asp Ile Ala Met Tyr Phe Cys Gln Gln Ser Arg Lys Ile
                85                  90                  95

Pro Tyr Thr Phe Gly Gly Gly Thr Lys Leu Glu Ile
            100                 105